# EUROPEAN PATENT APPLICATION

(11) **EP 1 147 996 A1**
(43) Date of publication of application: **24.10.2001**
(21) Application number: 00306455.7
(22) Date of filing: 28.07.2000
(51) Int. Cl.: B65D 83/08, A61B 19/04

(54) **Package for thin film products**

(30) Priority: 21.04.2000 CN 00104229
(71) Applicant: Chum, Tung Hang, Kwai Chung, New Territories, Hong Kong (CN)
(72) Inventor: Chum, Tung Hang, Kwai Chung, New Territories, Hong Kong (CN)
(74) Representative: Stevens, Jason Paul

(57) **Abstract**

A package for thin film products comprises a stack of thin film products (2a,2b,14) in a container. There are weak attachment points (14,4a,4b) between the thin film products, and an opening in the package is designed to resist retrieval of multiple products. When a thin film product is removed from the container, it draws the next product from the container as a result of the attachment between them. However, the attachment breaks before the second product is removed from the container, so that a part of the second product is left projecting from the container. The result is a package in which the leading edge of a single use portion of thin film product is always conveniently positioned to be grasped and removed.

## Description

This invention relates generally to the packaging of thin film products such as plastic bags or plastic films. This invention relates more particularly to a package and arrangement of thin film products which simplifies removal of a single use portion of thin film product.

Consumer products made of plastic films are widely used in the home and in industry. Plastic film products include plastic bags, plastic gloves, plastic wraps, decorative cellophane products, and the like. These products are used in particular with reference to food packaging, preparation and storage. Thin film products are notoriously difficult for the user to handle because they are limp, very thin and subject to electrostatic charges which make them stick together.

Many thin film products are currently wound around a paper tube core and sold in a rectangular box which is equipped with a cutting saw. The user must find an edge of the thin film, stretch out the desired portion, and manipulate the saw edge to cut the film. This process is undesirable because it requires two hands; it exposes the film to contamination from contact with the hands, cutting tool and package; the thin, limp, static charged film is difficult to handle; and the resulting length of film may be incorrect.

Other thin film products, such as plastic bags, are often packaged by placing many separate bags in a box. When the user attempts to retrieve a single bag, typically the bags stick together from electrostatic attraction. This has the undesirable effects of requiring the user to use two hands to separate the bags; handle more than one bag resulting in possible contamination; and replace the unwanted bags in the box, which takes time and often results in a jumbled mess of bags stuffed back in the package.

There is a need in the art for an arrangement of and package for thin film products which allows the user to quickly and easily retrieve a single use portion of thin film product.

An object of the preferred embodiments of the present invention is to provide a new and improved package for thin film products which allows the user to quickly and easily retrieve a single use portion of thin film product.

Another object of the preferred embodiments of the present invention is to provide a new and improved package for thin film products which permits retrieval of a single use portion of thin film product wherein only the portion of thin film product to be used is contacted by the user.

A package for thin film products in accordance with a first aspect of the present invention comprises an arrangement of single use portions (sheets, bags, pouches, gloves, etc.) of thin film product in which each portion is connected to the next by at least a single point. This arrangement is placed in a package having an opening through which the portions may be retrieved. Removal of the top portion brings with it the connected part of the following portion. The single point connection is purposely designed to be a weak connection which breaks easily. The result is that a part of the next portion of thin film product projects from the package after removal of the previous portion. The projecting part, which is preferably an edge or more preferably a corner, is easily grasped with one hand and removed for use.

This arrangement and packaging for thin film products has the benefits of always distributing a uniform length of product; allowing one-handed retrieval; and improving sanitation by eliminating contact with cutting tools and permitting retrieval where only the item to be used comes in contact with the user.

These and other objects, features and advantages of at least the preferred embodiments of the invention will become readily apparent to those skilled in the art upon reading the description of the preferred embodiments, in conjunction with the accompanying drawings, in which:-
Figure 1 is a perspective view, partly broken away, of an embodiment of a package for thin film products in accordance with the present invention; and
Figure 2 is a perspective view, partly broken away, of an alternative embodiment of a package in accordance with the present invention.

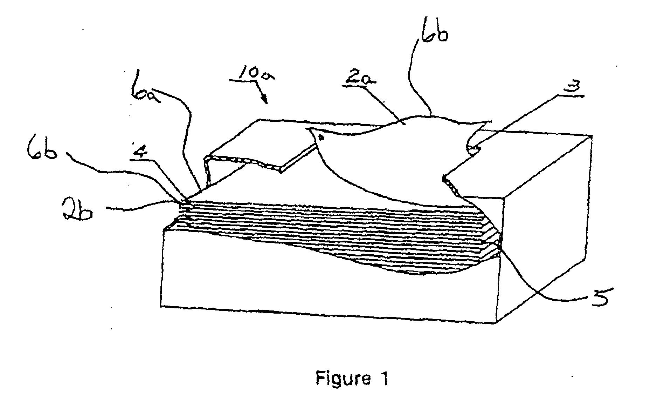

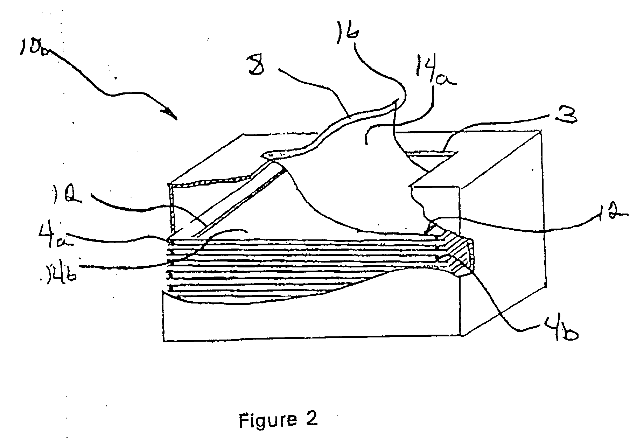

With reference to Figures 1 and 2, embodiments of a package for thin film products (referred to as a convenience package) in accordance with an aspect of the present invention are designated by the numerals 10a, 10b.

Figure 1 illustrates a convenience package 10a containing a stack of folded thin film sheets 2a, 2b. An opening 3 is provided in the top of the convenience package through which individual sheets 2a, 2b are retrieved. The sheets 2a, 2b are stacked with their free edges 6a, 6b to the left of the Figure and their folded edges 5 to the right. The trailing edge 6a of each sheet is attached to the leading edge 6b of the sheet below. The attachment point 4 between sheets 2a, 2b is of a strength sufficient only to pull the leading edge 6b through the opening 3. This accomplished, the attachment point 4 breaks, freeing the retrieved sheet for use.

The top sheet 2a in the stack is illustrated with its leading edge 6b protruding from the opening 3. To remove the protruding sheet 2a for use, the user simply grasps the protruding portion and pulls the sheet 2a. As the sheet 2a emerges from the opening 3, the leading edge 6b of the following sheet 2b is pulled through the opening by the attachment point 4. Thus, a sheet is always positioned for quick and easy retrieval.

The embodiment of the convenience package 10a illustrated in Figure 1 has several attributes to recommend it. Sheets are easily retrieved with one hand, freeing the other hand for other tasks. Only the sheet being used is touched and that sheet contacts only the user's hand and the inside edges of the convenience package 10a. The retrieved sheet is opened during retrieval and immediately ready for use. The folded configuration of the sheets allows all of the attachment points 4 to be positioned along one side of the stack while reducing the footprint of the convenience package 10a to at least half the dimension of the open sheets.

Figure 2 illustrates an alternative embodiment of a convenience package 10b. This convenience package contains a stack of unfolded zipper type plastic bags 14. Because the bags 14 are not folded, the attachment points 4a, 4b must alternate between the left and right sides of the convenience package 10b. The bags 14 are stacked with their zipper ends 12 staggered. (This arrangement creates a more uniform stack than if all the zippers were at one end.) Typically, the zipper end 12 of such bags has a strip of relatively unused plastic on which the attachment point 4a, 4b may be conveniently located. The opposed, bottom end 8 of the zipper bag 14 typically has no such extra plastic. In the illustrated embodiment 10b, the bottom seal 16 of the zipper bags 14 is positioned to leave a strip of plastic for use as an attachment point 4a, 4b. (The concern here is that breaking an attachment point formed above the bottom seal may weaken or rupture the bag.)

Thus, in the convenience package 10b, the zipper edge 12 of zipper bag 14a is connected via attachment point 4b to the bottom end 8 of the zipper bag 14b below. The top zipper bag 14a in the stack is illustrated with its bottom end 8 protruding from the opening 3. The zipper bag 14a may be grasped and removed, attachment point 4b drawing the bottom end 8 of the next bag 14b through the opening 3. The size and shape of the opening in the convenience package will vary depending upon the size, shape and configuration of the thin film material being packaged. The opening should allow the protruding sheet to be easily retrieved while exerting sufficient resistance on the following sheet to cause the attachment point to separate. The opening configuration for convenience package 10b, for example, may be influenced by the trailing zipper edge 12, which is typically substantially stiffer than the bottom edge 8 of such bags. The protruding zipper bag 14a will bring its zipper end 12, while the zipper end 12 of the following bag 14b will resist passing through the opening 3. The resistance of the zipper end 12 is sufficient to break the attachment point 4b, leaving the bottom end 8 of zipper bag 14b protruding from the opening 3.

In an alternative form, the zipper bags can be arranged in a stack such that all of the zipper ends are on the same side of the container (corresponding to the front of the container as shown in Figure 2), and overlie each other. This has the advantage that all of the attachment points can be formed in the strip of unused plastic above the zipper, which reduces the risk of weakening or rupturing the bag as it is removed from the container.

It should be noted that the attachment point can be accomplished using a variety of techniques. Heat bonding recommends itself due to the properties of materials used to manufacture many thin film products. Heat bonding has the advantage of simplicity and adds no adhesive or other foreign materials to the product. However, any adhesive or other connecting method may be used, assuming of course that the connecting method is compatible with the end use intended for the thin film product. For example, adhesive may be more appropriate for connecting plastic gloves than for items used in food storage.

Likewise the shape, location and size of the attachment point may vary substantially without departing from the scope of the present invention. The positioning of the attachment point will influence which part of the thin film product will be drawn through the opening and remain to be grasped. Multiple attachment points are also possible. A key aspect of the attachment point, whatever the position or configuration, is that it should only be strong enough to pull a part of the thin film product through the opening prior to separation.

While preferred embodiments of the foregoing invention have been set forth for purposes of illustration, the foregoing description should not be deemed a limitation of the invention herein. Accordingly, various modifications, adaptations and alternatives may occur to one skilled in the art without departing from the scope of the present invention.

## Claims

1. A package for thin film products, said package comprising:
a stack of individual thin film products (2a,2b, 14), each said individual thin film product including at least one attachment point (4,4a,4b), a leading portion (6b) and a trailing portion (6a); and
a container having an upper surface, said upper surface including an opening (3) ;
wherein said individual thin film products (2a,2b, 14) are serially connected to each other, said stack is disposed in said container and said individual thin film products are sequentially removable through said opening (3), each said attachment point (4,4a,4b) being positioned such that removal of a first individual thin film product through said opening causes a leading portion of an immediately following second individual thin film product to protrude from said opening, each said attachment point further being configured to separate whereby said leading portion remains protruding from said opening.

2. A package as claimed in claim 1, wherein each said individual thin film product (2a,2b,14) comprises a trailing portion and at least two attachment points (4, 4a,4b), said attachment points being positioned to connect the trailing portion of each individual thin film product to the leading portion of an immediately following individual thin film product.

3. A package as claimed in claim 1 or claim 2, wherein each said individual thin film product (2a,2b) is folded.

4. A package as claimed in claim 3, wherein removal of said thin film product (2a,2b) through said opening unfolds said thin film product.

5. A package as claimed in claim 3, wherein each said at least one attachment point is generally aligned above or below another at least one attachment point.

6. A package as claimed in claim 1 or claim 2, wherein each individual thin film product (14) comprises a trailing edge and at least first and second attachment points, said first attachment point being adjacent to said leading edge and said second attachment point being adjacent to said trailing edge.

7. A package as claimed in any of claims 1 to 6, wherein said individual thin film products comprise sheets of plastic film.

8. A package as claimed in any of claims 1 to 6, wherein said individual thin film products comprise plastic bags.

9. A package as claimed in any of claims 1 to 6, wherein said individual thin film products comprise plastic gloves.

10. A package as claimed in any preceding claim, wherein said opening is configured to permit the retrieval of a single individual thin film product (2a, 2b,14) and resist retrieval of multiple individual thin film products.
